# EUROPEAN PATENT SPECIFICATION

(11) **EP 0 934 045 B1**
(45) Date of publication and mention of the grant of the patent: **11.04.2001**
(21) Application number: 97910666.3
(22) Date of filing: 17.10.1997
(51) Int. Cl.: A61F 13/15, A61F 13/56

(54) **ABSORBENT ARTICLE HAVING THE ABILITY TO RESIST DEFORMATION**
ABSORBIERENDER ARTIKEL, FÄHIG VERFORMUNG ZU WIDERSTEHEN
ARTICLE ABSORBANT CAPABLE DE RESISTER A LA DEFORMATION

(30) Priority: 24.10.1996 SE 9603885
(43) Date of publication of application: 11.08.1999
(73) Proprietor: SCA Hygiene Products AB, 405 03 Göteborg (SE)
(72) Inventor: HANSSON, Roy, S-431 50 Mölndal (SE); DREVIK, Solgun, S-435 35 Mölnlycke (SE)
(74) Representative: Romare, Laila Anette
(86) International application number: SE9701741
(87) International publication number: WO9817218

(56) References cited:
- EP-A- 0 393 953
- EP-A- 0 607 986
- US-A- 4 690 680
- US-A- 4 886 513

## Description

### TECHNICAL FIELD:

The invention relates to an absorbent article such as an incontinence guard or a sanitary napkin, comprising an absorbent body enclosed between a liquid-pervious cover layer and a liquid-impervious cover layer, wherein the article has a substantially elongate shape and exhibits two end portions and a crotch portion arranged between the end portions.

### BACKGROUND OF THE INVENTION:

Absorbent articles of this type are intended to be worn by individuals with relatively light incontinence problems, or by menstruating women, something which puts high demands on the articles being comfortable to wear, at the same time as they should be leakproof.

When using such an absorbent article, however, one problem has proved to be that it is difficult to maintain the shape during use. The most important reason for this is that the article is deformed during use by the forces which arise when the user moves. The deformation implies that the article is creased, something which gives the user an uncomfortable and chafing sensation, at the same time as it increases the risk of leakage and the article becomes more discernible under the clothing of the user.

The fact that deformation of absorbent articles during use causes problems in the form of an increased risk of leakage, has long been known. However, previous attempts to solve this problem have aimed at minimizing the risk of leakage of an absorbent article which by means of deformation during use already has obtained a creased shape.

For instance, it is previously known through SE 469 621 to provide an absorbent article, such as a sanitary napkin or an incontinence guard, with raised liquid barriers along the side edges of the article. One disadvantage with the raised liquid barriers, however, is that the liquid barriers risk losing their function when the article is subjected to deformation since a prerequisite for the barrier function is that the barrier flaps remain in a raised condition during use.

Furthermore, it is previously known through SE 455 668, US 4 285 343, EP 130 848, EP 134 086 and US 4 608 047 to provide sanitary napkins with pliable side flaps or wings, projecting from the absorbent body. One disadvantage with the side flaps, however, is that folds often arise in which liquid may flow out onto one of the side flaps when the article is deformed. Since the side flaps normally do not have the capacity to absorb more than a small quantity of liquid, there is a risk that the liquid will flow out to the panties of the user.

Still another disadvantage with side flaps is that they usually are provided with an adhesive attachment member in order to make it possible to attach them to the underlying panties. Such an attachment member requires that the adhesive is protected from direct contact with adjacent material before use. This may be achieved, for example, by arranging a detachable protective layer over the attachment device, or by means of a special folding of the article.

In an article disclosed in the Swedish Patent Application SE 9502747 (& GB-A-2 303 821) the need for a detachable protective layer on the side flaps has been eliminated, by means of an attachment member exhibiting a self-adhesive, pressure-sensitive adhesive layer, further comprising an essentially incompressible, non-adhesive cover layer, e.g. a thin nonwoven arranged over the adhesive layer. Thereby, the cover layer exhibits a plurality of apertures or pores, substantially arranged perpendicularly to the plane of the cover layer, through which the attachment surface of the adhesive layer is exposed so that the attachment device is attachable against textile or textile-like material surfaces.

Another pressure-sensitive adhesive attachment device is disclosed in EP 393,953. The attachment device comprises a carrier, one surface of which is provided with a pressure-sensitive adhesive applied in the spaces between a row of spines or protuberances projecting in a direction from the carrier, whereby the spines or the protuberances extend out past the adhesive. The attachment device is particularly suited for use against textile fabric, whereby the spines or protuberances are able to penetrate down through the surface of the textile fabric so that the adhesive is brought into contact with, and adheres to, the textile fabric. However, a problem with placing such a pressure-sensitive adhesive attachment device on the side flaps may be that the side flaps obtain a considerably increased stiffness. Since the side flaps often are placed so that they attach to the outside of the crotch portion of the panties during use, the side flaps may come to be in contact against the thighs of the user and thereby may be perceived as chafing and uncomfortable.

US 4,690,680 and EP 607,986 disclose absorbent articles having conventional fastening adhesive applied thereto.

US 4,886,513 discloses an absorbent article having a reinforcing member.

A further disadvantage, which mainly is associated with incontinence guards and other absorbent articles which are worn by elderly people or handicapped persons with reduced ability to move their hands, is the very placing of the article in the panties. The absorbent articles of today are usually attached to the panties by means of a self-adhesive glue which is protected by a detachable protective layer when the article is not in use. Thereby, one problem is that the article is easily creased or deformed in another way when placing the article in the panties. This may cause glue to come into contact with glue. Since it is difficult to release the contact areas of the glue from each other, without destroying the article, such an article often becomes useless and has to be discarded. If an article in such a deformed state is nevertheless used, there is a large risk of leakage.

### SUMMARY OF THE INVENTION:

The problem that the absorbent article is subjected to deformation and thereby creasing during use, which creates an uncomfortable, chafing sensation, at the same time as an increased risk of leakage arises, may be reduced by means of the present invention.

Furthermore, by means of the present invention, the risk of the article being deformed when actually placing it in the panties, causing glue to get into contact with glue, is minimized.

An article designed according to the invention is primarily characterized in that at least one of the end portions of the article exhibits a higher stiffness than the crotch portion of the article. The stiffness is at least 15% higher in the end portion/end portions that in the crotch portion. Since the article exhibits a higher stiffness at the end portion, the fit and the shape stability are improved, without the crotch portion of the article becoming stiff and uncomfortable.

According to a preferred embodiment, the stiffness in the end portion is approximately 25% higher than the stiffness in the crotch portion of the article.

According to one embodiment, a stiffening layer is attached on the side of the liquid-impervious cover layer which is facing away from the absorbent body. An advantage with such an embodiment is that the stiffening layer thereby may be completely or partially constituted by some kind of attachment device, intended for attachment of the article to the panties of the user. The stiffening portion may, for example, be constituted by an adhesive such as, for example, PSHM, which stands for "pressure sensitive hot melt", i.e. what is commonly referred to as hot melt adhesive, a tape, or a similar adhesive attachment device suitable for the invention. Furthermore, the stiffening portion may be constituted by a mechanical attachment system, comprising a material which, at the same time as it acts as a stiffener in the end portion, also exhibits a high friction against the underlying panties.

According to another embodiment, the stiffening layer is attached on the side of the liquid-impervious cover layer which is facing the absorbent body. According to this embodiment, the stiffening layer may also comprise a part of the absorbent body, and consist of, for example, pulp fibres or another material suitable for the purpose of the invention. Furthermore, the higher stiffness may be obtained by means of the absorbent body being more compressed in the end portions of the article than in the crotch portion.

According to still another embodiment, a stiffening layer is attached to each of the two end portions. An advantage with such an embodiment is that the risk of the article being deformed during attachment in the panties or during use is almost completely eliminated.

According to still another embodiment, the stiffening layer has the same or a smaller extension than the absorbent body at the end portions. This implies that the outermost edge on the absorbent article lacks a stiffening layer and consequently exhibits the same softness as the crotch portion of the article. Thereby, the risk that the stiffening portions are perceived as being chafing is minimized.

Furthermore, according to still another embodiment, the stiffened end portions consist of a pressure-sensitive adhesive attachment device which comprises an essentially incompressible, non-adhesive cover layer with a thickness which preferably does not exceed 0.5 mm and wherein the mutual distance between two adjacent openings or pores in the cover layer is smaller than 3 mm. The cover layer exhibits a plurality of through apertures or pores, essentially arranged perpendicularly to the plane of the cover layer, and is applied over and attached to the attachment surface of the adhesive layer. The adhesive attachment device is intended to interact with a textile or a textile-like receiving surface.

Another useful pressure-sensitive adhesive attachment device comprises a carrier, one side of which is provided with a pressure-sensitive adhesive applied in the spaces between a number of spines or protuberances projecting from the carrier, the spines or protuberances projecting out past the adhesive.

### BRIEF DESCRIPTION OF THE DRAWINGS:

In the following the invention will be described in greater detail, with reference to the embodiments which are shown in the attached drawings.

Fig. 1 shows an incontinence guard according to the invention, seen from the side which is intended to be facing away from the user during use.

Fig. 2 shows a sanitary napkin according to the invention, seen from the side which is intended to be facing away from the user during use.

Fig. 3 shows another incontinence guard according to the invention, seen from the side which is intended to be facing away from the user during use.

Fig. 4 shows still another sanitary napkin according to the invention, seen from the side which is intended to be facing away from the user during use.

### DETAILED DESCRIPTION OF PREFERRED EMBODIMENTS:

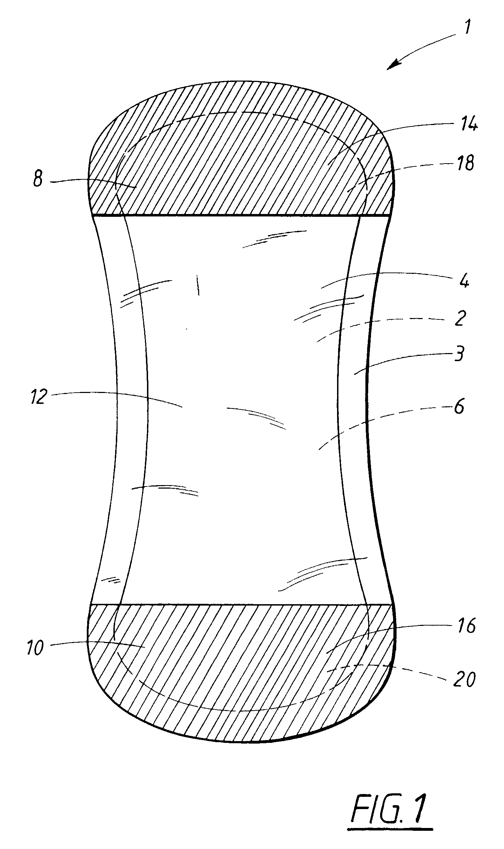

The sanitary napkin 1 shown in Fig. 1 comprises a first liquid-pervious cover layer 2, arranged over the surface on the incontinence guard 1 which is intended to be facing the user during use, and a second, liquid-impervious cover layer 4 arranged over the surface of the article which is intended to be facing away from the user during use. An absorbent body 6 is enclosed between the two cover layers 2, 4. The incontinence guard 1 is hourglass-shaped and exhibits two wider end portions 8, 10 and a narrower crotch portion 12 located between the end portions 8, 10. The crotch portion 12 is intended to be located at the narrowest region between the thighs of the user during use, and is the region of the absorbent body which is expected to be wetted first by excreted body fluid.

The cover layers 2, 4 have a larger extension in the plane of the incontinence guard 1 than the absorbent body 6, around its entire periphery. The projecting portions 3 of the cover layers 2, 4 are mutually connected around the absorbent body 6, for example by means of gluing, welding, or in another way. Stiffening layers 14, 16 are connected to the liquid-impervious cover layer 2 at the end portions 8, 10. The stiffening layers 14, 16 extend from the each respective end edge, at the most one third of the entire length of the incontinence guard 1, and are connected to the liquid-impervious cover layer 4 by gluing, welding, or in another way.

The liquid-pervious cover layer 2 may consist of any material suitable for the purpose. Examples of commonly occurring liquid-pervious cover materials are non-woven textile materials, so-called nonwoven materials, perforated plastic films, scrims of plastic or textile and liquid-pervious foam layers.

The liquid-impervious cover layer 4 may also consist of a number of different materials. Most commonly, the liquid-impervious cover layer 4 is constituted by a thin liquid-impervious plastic film. However, it is also possible to utilize other types of liquid-impervious materials, such as nonwoven materials which have been made liquid-impervious, for example by plastic coating, liquid-impervious foam layers, liquid-impervious adhesive or the like.

The absorbent body 6 may, for example, be constituted by one or several layers of cellulose fluff pulp. Thereby, the cellulose fluff pulp may be blended with fibres or particles of a highly absorbent polymeric material of the type which during absorption chemically binds large quantities of liquid, during the formation of a liquid-containing gel. Furthermore, additional components may be included in the absorbent body in order to improve the properties of the absorbent body 6. Examples of such components are binder fibres, different types of liquid-distributing layers or fibres, shape-stabilizing components, or the like. Naturally, the absorbent body 6 may consist of other types of absorbent materials, such as absorbent nonwoven materials, absorbent foams, or mixtures of different sorts of absorbent materials.

The stiffening layers 14, 16 may consist of any material suitable for the purpose which gives the end portions 8, 10 of the incontinence guard 1 a stiffness increase of at least 15% in comparison to the remaining portions of the incontinence guard. Preferably, the end portions 8, 10 of the incontinence guard exhibit a stiffness which is approximately 25% higher that the portions of the incontinence guard which are without stiffening material. In order to measure the stiffness of the different portions the ASTM-method D 4032.82 CIRCULAR BEND PROCEDURE is utilized, which method is thoroughly described in EP 0,336,578. The equipment consists of a rectangular plate which has a circular, funnel-shaped opening in the middle. The material is placed over the circular opening and is loaded with a force so that the material reaches the bottom of the plate. The force is measured and, accordingly, provides a measure of the stiffness of the material.

The stiffening layers 14, 16 may, for example, be constituted by an adhesive attachment device which exhibits an adhesive capability against textile surfaces. Such an adhesive attachment device may comprise a carrier, one side of which is provided with a pressure-sensitive adhesive which is applied in the spaces between a row of spines or protuberances projecting from the carrier, whereby the spines or protuberances project out past the adhesive. Another attachment device comprises a pressure-sensitive adhesive layer, characterized in that an essentially incompressible, non-adhesive cover layer, exhibiting a plurality of through apertures or pores which are substantially arranged perpendicularly to the plane of the cover layer, is applied across and attached to the attachment surface 18, 20 of the adhesive layer. The thickness of the cover layer may be varied depending on the desired stiffening degree in the end portions 8, 10 of the article, but preferably the thickness does not exceed 0.5 mm, However, it is also possible to use other types of stiffening adhesives, for example of hot melt type, or other stiffening materials such as a plastic film, a nonwoven material, a foam material or the like.

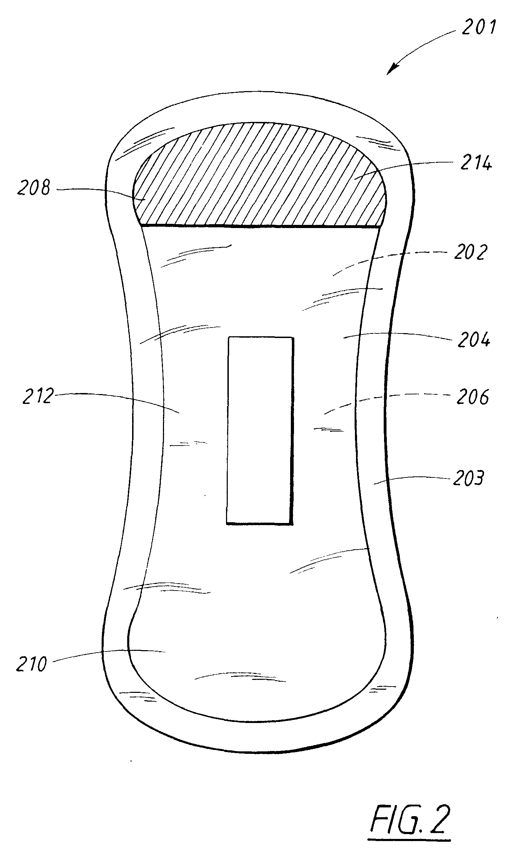

The sanitary napkin 201 shown in Fig. 2 generally has the same construction as the incontinence guard 1 in Fig. 1. Accordingly, the sanitary napkin 201 exhibits an absorbent body 206 enclosed between a first liquid-pervious cover layer 202 and a liquid-impervious cover layer 204. The sanitary napkin 201 exhibits a front end portion 208, and a back end portion 210, and also a crotch portion 212 located between the end portions 208, 210. The front end portion 208 is wider in the transverse direction than the crotch portion 212 and the back end portion 210.

The cover layers 202, 204 have a larger extension in the plane of the sanitary napkin 201 than the absorbent body 206, around the entire periphery of this. The projecting portions 203 of the cover layers 202, 204 are mutually connected around the absorbent body 206, for example by means of gluing, welding, or in another way.

A stiffening layer 214 is connected to the liquid-impervious cover layer 204 at the end portion 208. The stiffening layer 214 follows the contours of the absorbent body in the end portion, implying that the projecting portion 203 of the end portion, which is formed by cover layers 202, 204, remains soft. The stiffening layer 214 is connected to the liquid-impervious cover layer 204, for example by gluing, welding, or in another way.

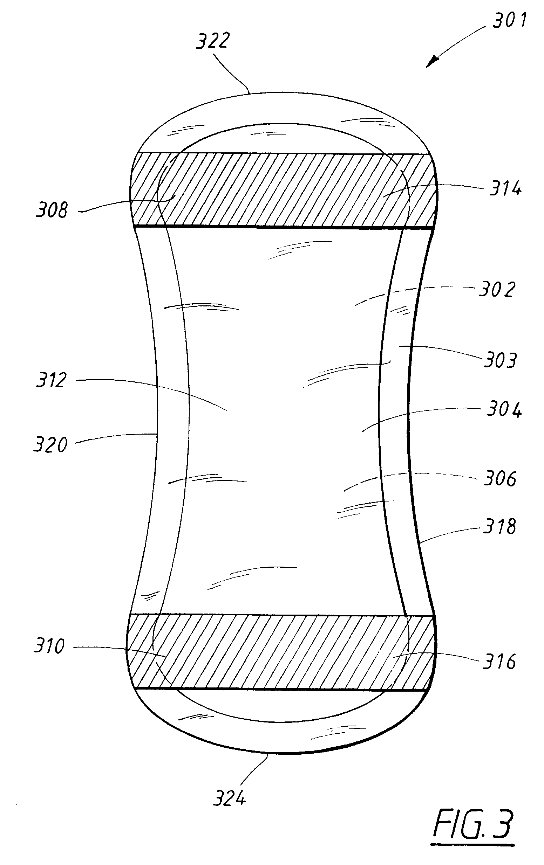

In Fig. 3 still another incontinence guard 301 is shown, comprising a first liquid-pervious cover layer 302, a second liquid-impervious cover layer 304, and an absorbent body 306 enclosed between the cover layers 302, 304. As in the previously described incontinence guard 1, the absorbent body 306 of the incontinence guard 301 is hourglass-shaped, with wider end portions 308, 310 and narrower crotch portion 312. Furthermore, the incontinence guard 301 exhibits two longitudinal concavely-curved side edges 318, 320 and two transverse convexly-curved end edges 322, 324. The two cover layers 302, 304 have the same shape as the absorbent body 306, but a slightly larger extension in the plane of the incontinence guard 301, and are mutually connected within the projecting portions 303 of the cover layers 302, 304. The incontinence guard 301 exhibits two stiffening layers 314, 316 which are constituted by two transverse strips connected to the liquid-impervious cover layer 304 a distance from the respective end edges 322, 324.

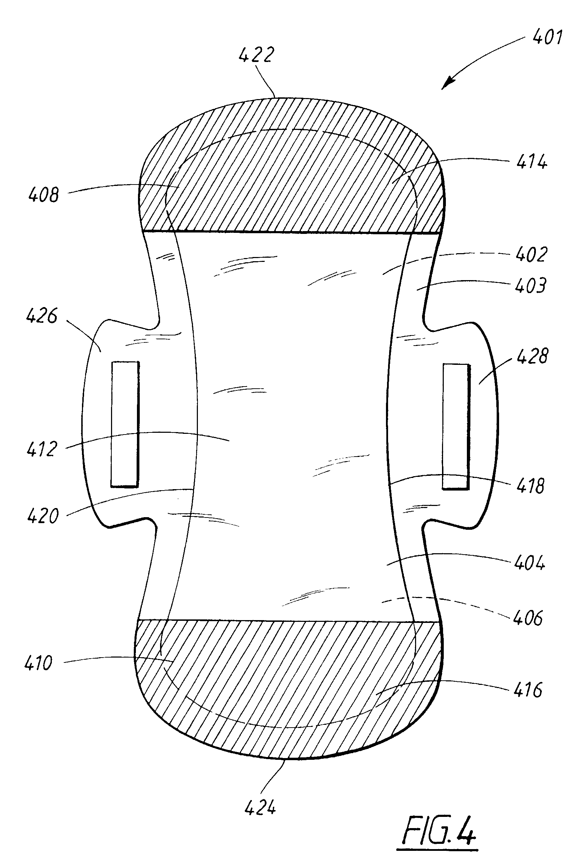

In Fig. 4 another sanitary napkin 401 is shown, comprising a first liquid-pervious cover layer 402, a second liquid-impervious cover layer 404, and an absorbent body 406 enclosed between the cover layers 402, 404.

The absorbent body 404 is hourglass-shaped with wider end portions 408, 410 and a narrower crotch portion 412. Furthermore, the absorbent body 406 has two longitudinal concavely-curved side edges 418, 420 and two transverse convexly-curved end edges 422, 424. The two cover layers 402, 404 have a slightly larger extension than the absorbent body 406 in the plane of the sanitary napkin 401 and the projecting portions 403 of the cover layers 402, 404 are mutually connected and form a continuous edge around the absorbent body 406.

At the crotch portion 412 of the absorbent body 406, a side flap 426, 428 projects out from each longitudinal side edge 418, 420. The side flaps 426, 428 are formed out of portions of the cover layers 402, 404 and each side flap 426, 428 consists of a layer of liquid-pervious material and a layer of liquid-impervious material. During the use of the sanitary napkin 401, the side flaps 426, 428 are intended to be able to be folded around the leg edges of the panties of the user. Stiffening layers 414, 416 are connected to the liquid-impervious cover layer 404 at the end portions 408, 410.

The invention also includes all conceivable combinations of the described embodiments.

## Claims

1. An absorbent article, such as an incontinence guard or a sanitary napkin, comprising an absorbent body (6) enclosed between a liquid-pervious cover layer (2) and a liquid-impervious cover layer (4), wherein the article has a substantially elongate shape and exhibits two end portions (8, 10), and a crotch portion (12) arranged between the end portions,
**characterized in** that at least one of the end portions (8, 10) of the article exhibits a stiffness as measured according to ASTM D 4032-82 which is at least 15% higher than the stiffness in the crotch portion (12) of the article.

2. An absorbent article according to claim 1,
**characterized in** that at least one of the end portions (8, 10) of the article exhibits a stiffness which is at least 25% higher than the stiffness in the crotch portion (12) of the article.

3. An absorbent article according to claim 1 or 2,
**characterized in** that a stiffening layer (14, 16) is attached to the liquid-impervious cover layer (4) at at least one of the end portions (8, 10), whereby the end portion (8, 10) exhibits higher stiffness than the crotch portion (12) of the article.

4. An absorbent article according to claim 3,
**characterized in** that the stiffening layer (14, 16) is attached on the side of the liquid-impervious cover layer (4) which is facing away from the absorbent body (6).

5. An absorbent article according to claim 3,
**characterized in** that the stiffening layer (14, 16) is attached on the side of the liquid-impervious cover layer (4) which is facing the absorbent body (6).

6. An absorbent article according to any one of the preceding claims, **characterized in** that a stiffening layer (14, 16) is attached to each end portion (8, 10).

7. An absorbent article according to any one of the preceding claims, **characterized in** that the stiffening layer (14, 16) has the same or a smaller extension than the absorbent body (6) at the end portions (8, 10).

8. An absorbent article according to any one of the preceding claims, **characterized in** that the stiffened end portions (8, 10) comprise a pressure-sensitive adhesive attachment device.

9. An absorbent article according to claim 8,
**characterized in** that the pressure-sensitive adhesive attachment device comprises an essentially incompressible, non-adhesive cover layer exhibiting a plurality of through openings or pores, arranged substantially perpendicularly to the plane of the cover layer, which is applied across and attached to the attachment surface of the adhesive layer (18, 20) whereby the attachment device is intended to interact with a textile or textile-like receiving surface.

10. An absorbent article according to claim 9,
**characterized in** that the thickness of the essentially incompressible, non-adhesive cover layer is smaller than 0.5 mm and that the mutual distance between two adjacent openings or pores in the cover layer is smaller than 3 mm.

11. An absorbent article according to claim 8,
**characterized in** that the pressure-sensitive adhesive attachment device comprises a carrier, one side of which is provided with a pressure-sensitive adhesive.

12. An absorbent article according to claim 11,
**characterized in** that the pressure-sensitive adhesive is applied in the spaces between a plurality of spines or protuberances projecting from the carrier, whereby the spines or protuberances extend out past the adhesive.

## Patentansprüche

1. Absorptionsartikel, wie z.B. Inkontinenzschutz oder Hygienebinde, mit einem Absorptionskörper (6), der zwischen einer flüssigkeitsdurchlässigen Deckschicht (2) und einer flüssigkeitsundurchlässigen Deckschicht (4) eingeschlossen ist, wobei der Artikel eine im Wesentlichen längliche Form aufweist und zwei Endabschnitte (8, 10) und einen Schrittabschnitt (12) aufweist, der zwischen den Endabschnitten angeordnet ist,
dadurch **gekennzeichnet**, dass wenigstens einer der Endabschnitte (8, 10) des Artikels eine Steifigkeit, gemessen gemäß ASTM D 4032-82 aufweist, die wenigstens 15% höher als die Steifigkeit in dem Schrittabschnitt (12) des Artikels ist.

2. Absorptionsartikel nach Anspruch 1,
dadurch **gekennzeichnet**, dass wenigstens einer der Endabschnitte (8, 10) des Artikels eine Steifigkeit aufweist, die wenigstens 25% höher als die Steifigkeit in dem Schrittabschnitt des Artikels ist.

3. Absorptionsartikel nach Anspruch 1 oder 2,
dadurch **gekennzeichnet**, dass
eine Versteifungsschicht (14, 16) an die flüssigkeitsundurchlässige Deckschicht (4) an wenigstens einem der Endabschnitte (8, 10) angebracht ist, wodurch der Endabschnitt (8, 10) eine höhere Steifigkeit als der Schrittabschnitt (12) des Artikels aufweist.

4. Absorptionsartikel nach Anspruch 3,
dadurch **gekennzeichnet**, dass
die Versteifungsschicht (14, 16) an die Seite der flüssigkeitsundurchlässigen Deckschicht (4) angebracht ist, die von dem Absorptionskörper (6) weggerichtet ist.

5. Absorptionsartikel nach Anspruch 3,
dadurch **gekennzeichnet**, dass
die Versteifungsschicht (14, 16) an die Seite der flüssigkeitsundurchlässigen Deckschicht (4) angebracht ist, die zu dem Absorptionskörper (6) gerichtet ist.

6. Absorptionsartikel nach einem der vorangehenden Ansprüche,
dadurch **gekennzeichnet**, dass eine Versteifungsschicht (14, 16) an jedem Endabschnitt (8, 10) angebracht ist.

7. Absorptionsartikel nach einem der vorangehenden Ansprüche,
dadurch **gekennzeichnet**, dass die Versteifungsschicht (14, 16) die gleiche oder eine kleinere Ausdehnung als der Absorptionskörper (6) an den Endabschnitten (8, 10) aufweist.

8. Absorptionsartikel nach einem der vorangehenden Ansprüche,
dadurch **gekennzeichnet**, dass
die versteiften Endabschnitte (8, 10) eine selbsthaftende Haft-Anbringvorrichtung aufweisen.

9. Absorptionsartikel nach Anspruch 8,
dadurch **gekennzeichnet**, dass
die selbsthaftende Haft-Anbringvorrichtung eine im Wesentlichen nicht komprimierbare, nicht haftende Deckschicht mit mehreren Durchgangsöffnungen oder Poren aufweist, die im Wesentlichen senkrecht zu der Ebene der Deckschicht angeordnet sind, die über die Anbringfläche der Haftschicht (18, 20) aufgebracht ist und an diese angebracht ist, wodurch die Anbringvorrichtung dafür vorgesehen ist, mit einer textilen oder textilartigen Aufnahmefläche zusammenzuwirken.

10. Absorptionsartikel nach Anspruch 9,
dadurch **gekennzeichnet**, dass die Dicke der im Wesentlichen nicht komprimierbaren, nicht haftenden Deckschicht kleiner als 0,5 mm ist, und dass der gegenseitige Abstand zwischen zwei benachbarten Öffnungen oder Poren in der Deckschicht geringer als 3 mm ist.

11. Absorptionsartikel nach Anspruch 8,
dadurch **gekennzeichnet**, dass die selbsthaftende Haft-Anbringvorrichtung einen Träger aufweist, dessen eine Seite mit einem selbsthaftenden Haftmittel versehen ist.

12. Absorptionsartikel nach Anspruch 11,
dadurch **gekennzeichnet**, dass
das selbsthaftende Haftmittel in den Räumen zwischen mehreren Stacheln oder Ausstülpungen aufgebracht ist, die von dem Träger vorstehen, wobei sich die Stacheln oder Ausstülpungen über das Haftmittel hinaus nach außen erstrecken.

## Revendications

1. Article absorbant, tel qu'une protection contre l'incontinence ou une serviette hygiénique, comprenant un corps absorbant (6) enfermé entre une enveloppe (2) perméable aux liquides et une enveloppe (4) imperméable aux liquides, dans lequel l'article a une forme substantiellement allongée et présente deux parties d'extrémité (8, 10), et une partie d'entrejambes (12) agencée entre les parties d'extrémité, **caractérisé** en ce qu'au moins une des parties d'extrémité (8, 10) de l'article présente une rigidité, telle que mesurée selon la norme ASTM D 4032-82, qui est supérieure d'au moins 15 % à la rigidité de la partie d'entrejambes (12) de l'article.

2. Article absorbant selon la revendication 1, caractérisé en ce qu'au moins une des parties d'extrémité (8, 10) de l'article présente une rigidité qui est supérieure d'au moins 25 % à la rigidité de la partie d'entrejambes (12) de l'article.

3. Article absorbant selon la revendication 1 ou 2, caractérisé en ce qu'une couche de raidissement (14, 16) est attachée à l'enveloppe (4) imperméable aux liquides en au moins une des parties d'extrémité (8, 10), grâce à quoi la partie d'extrémité (8, 10) présente une plus grande rigidité que la partie d'entrejambes (12) de l'article.

4. Article absorbant selon la revendication 3, caractérisé en ce que la couche de raidissement (14, 16) est attachée sur la face de l'enveloppe (4) imperméable aux liquides qui tourne le dos au corps absorbant (6).

5. Article absorbant selon la revendication 3, caractérisé en ce que la couche de raidissement (14, 16) est attachée sur la face de l'enveloppe (4) imperméable aux liquides qui est tournée vers le corps absorbant (6).

6. Article absorbant selon l'une quelconque des revendications précédentes, caractérisé en ce qu'une couche de raidissement (14, 16) est attachée à chaque partie d'extrémité (8, 10).

7. Article absorbant selon l'une quelconque des revendications précédentes, caractérisé en ce que la couche de raidissement (14, 16) a une étendue identique ou inférieure à celle du corps absorbant (6) dans les parties d'extrémité (8, 10).

8. Article absorbant selon l'une quelconque des revendications précédentes, caractérisé en ce que les parties d'extrémité rigidifiées (8, 10) comprennent un dispositif de fixation adhésif sensible à la pression.

9. Article absorbant selon la revendication 8, caractérisé en ce que le dispositif de fixation adhésif sensible à la pression comprend une enveloppe non adhésive, globalement incompressible, présentant une pluralité de trous traversants ou pores, agencés sensiblement perpendiculairement au plan de l'enveloppe, qui est appliquée sur la largeur de la surface de fixation de la couche adhésive (18, 20), et attachée à cette dernière, le dispositif de fixation étant destiné à inter-agir avec une surface réceptrice textile ou de type textile.

10. Article absorbant selon la revendication 9, caractérisé en ce que l'épaisseur de l'enveloppe non adhésive et globalement incompressible est inférieure à 0,5 mm et en ce que la distance séparant deux ouvertures ou pores adjacent(e)s dans l'enveloppe est inférieure à 3 mm.

11. Article absorbant selon la revendication 8, caractérisé en ce que le dispositif de fixation adhésif sensible à la pression comprend un support, dont une face est munie d'un adhésif sensible à la pression.

12. Article absorbant selon la revendication 11, caractérisé en ce que l'adhésif sensible à la pression est appliqué dans les espaces situés entre des épines ou protubérances faisant saillie sur le support, les épines ou protubérances s'étendant plus loin que l'adhésif.
